(19)

**Europäisches Patentamt**
**European Patent Office**
**Office européen des brevets**

(11) **EP 2 717 774 B1**

(12) **EUROPEAN PATENT SPECIFICATION**

(45) Date of publication and mention
of the grant of the patent:
**15.11.2017 Bulletin 2017/46**

(51) Int Cl.:
*A61B 5/107* (2006.01)    *G02B 6/02* (2006.01)
*A61B 5/11* (2006.01)    *A61N 5/10* (2006.01)

(21) Application number: **12730040.8**

(22) Date of filing: **31.05.2012**

(86) International application number:
**PCT/IB2012/052753**

(87) International publication number:
**WO 2012/168836 (13.12.2012 Gazette 2012/50)**

(54) **DYNAMIC CONSTRAINING WITH OPTICAL SHAPE SENSING**

DYNAMISCHE EINSCHRÄNKUNG MIT OPTISCHER FORMMESSUNG

CONTRAINTE DYNAMIQUE COMPRENANT DÉTECTION OPTIQUE DE FORME

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **10.06.2011  US 201161495862 P
03.08.2011  US 201161514585 P**

(43) Date of publication of application:
**16.04.2014  Bulletin 2014/16**

(73) Proprietor: **Koninklijke Philips N.V.
5656 AE Eindhoven (NL)**

(72) Inventors:
• **MANZKE, Robert
5656 AE Eindhoven (NL)**
• **'T HOOFT, Gert ,Wim
5656 AE Eindhoven (NL)**
• **CHAN, Raymond
5656 AE Eindhoven (NL)**
• **DESJARDINS, Adrien, Emmanuel
5656 AE Eindhoven (NL)**

(74) Representative: **Steffen, Thomas
Philips Intellectual Property & Standards
High Tech Campus 5
5656 AE Eindhoven (NL)**

(56) References cited:
WO-A1-2011/048509    US-A1- 2005 244 094
US-A1- 2008 243 018    US-A1- 2009 180 666
US-A1- 2011 054 303    US-A1- 2011 090 486

EP 2 717 774 B1

## Description

This disclosure relates to optical shape sensing and more particularly to systems with optical shape sensing constraints applied to spatially disposed sensors arrays for medical applications.

Solutions to nonlinear and ill-posed problems such as electrical impedance tomography (EIT), diffuse optical tomography (DOT), body surface potential mapping (BSPM), etc. are typically approached using numerical methods which exploit prior knowledge about the measurements in relation to the unknown parameters of interest. Examples include finite element modeling for forward calculations and regularized nonlinear solvers for obtaining unique and stable inverse solutions. Typical scenarios encountered in industrial and medical imaging problems are fundamentally three-dimensional for time-invariant cases and four-dimensional for dynamic observations.

For arriving at meaningful inversion solutions, it is important to know the source / detector geometry of the experimental setup as well as the three-dimensional (3D) shape of the object / volume of interest. In the case of DOT, an input signal decays exponentially with distance from the source. A measurement setup adaptable to an object's geometry, would be preferable over a rigid, oversized one for generic use. In the case of EIT, the electrodes need to be in contact with tissue and dynamics due to a patient's movement, such as, breathing motion, need to be accounted for within the measurement geometry. The accuracy of an image reconstruction depends on how a 3D shape changes in time.

Currently, examples of additional prior knowledge being incorporated into the solution of inverse problems include imaging data from computed tomography (CT) or magnetic resonance (MR) for non-invasive electro-anatomical mapping / Body Surface Potential Mapping (BSPM), EIT and DOT. However, these datasets are typically acquired at a prior point in time and do not reflect the dynamics present within the structure of interest during measurement acquisition for tomographic inversion with non-contact mapping / EIT / DOT.

External beam radiotherapy (XRT) is delivered via two-dimensional beams using linear accelerator machines. XRT mainly consists of a single beam of radiation delivered to the patient from several directions: often front or back, and both sides. The treatment is planned or simulated on a specially calibrated diagnostic x-ray machine known as a simulator because the simulator recreates the linear accelerator actions, and compares them to the usually well-established arrangements of the radiation beams to achieve a desired plan. The aim of simulation is to accurately target or localize the volume which is to be treated. XRT benefits from knowledge of organ deformation during planning and execution.

Shape information can be derived from a variety of systems. These include: optical shape interrogation systems (e.g., fiber optic Bragg sensors, Rayleigh scattering, Brillouin scatter, optical intensity-based attenuation), multi-coil arrays for electromagnetic (EM) localization of points on the apparatus, laser scanning systems for three-dimensional surface estimation and optical/acoustic marker/emitter arrays for camera (time-of-flight or conventional optical measurement) or microphone-based interrogation of shape. Real-time imaging such as ultrasound may also be used for shape information, but the clinical viability of that approach depends on additional cost and clinical value of the tomographic information relative to the imaging performed.

US 2011/0054303 A1 discloses a device for generating a frame of reference and tracking the position and orientation of a tool in computer-assisted image guided surgery or therapy system.

A first curvature sensor including fiducial markers is provided for positioning on a patient prior to volumetric imaging, and sensing the patient's body position during surgery. A second curvature sensor is coupled at one end to the first curvature sensor and at the other end to a tool to inform the computer-assisted image guided surgery or therapy system of the position and orientation of the tool with respect to the patient's body.

In accordance with the present principles, a system for measuring dynamic movement of a subject include a mesh configured to flexibly and snuggly fit over at least a portion of the subject. The mesh includes one or more shape sensing optical fibers disposed therein and arranged to provide light reflected back from within the fibers, and a second modality of measurement other than optical fiber sensing that includes sensors or detectors incorporated and distributed within a throughout the mesh such that the one or more shape sensing optical fibers monitor movement of the sensors or detectors on a subject. A reconstruction module is coupled to the shape sensing fibers to receive feedback signals based on the reflected light from the shape sensing fibers and to interpret dynamic changes in a shape and position of the sensors or detectors based on the feedback signals. The reconstruction module accounts for the dynamic changes to improve a medical activity on the subject.

These and other objects, features and advantages of the present disclosure will become apparent from the following detailed description of illustrative embodiments thereof, which is to be read in connection with the accompanying drawings.

This disclosure will present in detail the following description of preferred embodiments with reference to the following figures wherein:

FIG. 1 is a block diagram showing an illustrative system which measures and accounts for dynamic changes in a patient during a procedure in accordance with the present principles;

Fig. 2 is a schematic diagram showing a system for image reconstruction using multiple position sensing technologies in accordance with one illustrative invention;

FIG. 3 is a diagram showing a mesh garment or a similar flexible tracked manifold / surface in accordance with one illustrative embodiment;

FIG. 4 is a diagram showing a radiation treatment setup being controlled in accordance with measured dynamic changes in accordance with an illustrative embodiment; and

FIG. 5 is a flow diagram showing steps for measuring and accounting for dynamic changes in a patient during a procedure in accordance with using the system of the present disclosure.

In accordance with the present principles, a measurement system and method provide an adaptable and optimized setup specifically configured for an object/geometry of interest. In particularly useful embodiments, shape sensing technology is employed to determine shape and locus information of a source-detector setup for ill-posed reconstruction problems. This helps to model and solve the ill-posed problem for adaptive and dynamic measurement setups.

In a particularly useful embodiment, one or more shape sensing optical fibers are employed in a mesh with electrical, thermal or other measurement sensors. The fibers and other sensors work in conjunction to provide superior results. For example, fiber positioning feedback from the sensors provides more accurate sensor positions to enable more accurate results for a plurality of different procedures or studies, such as, e.g., electrical impedance tomography, body surface potential / temperature mapping, etc. The optical shape sensing fibers may provide additional information to create a more complete picture for solving ill-posed problems.

It should be understood that the present invention will be described in terms of medical instruments; however, the teachings of the present invention are much broader and are applicable to any instruments employed in tracking or analyzing complex biological or mechanical systems. In particular, the present principles are applicable to internal tracking procedures of biological systems, procedures in all areas of the body such as the lungs, gastrointestinal tract, excretory organs, blood vessels, etc. The elements depicted in the FIGS. may be implemented in various combinations of hardware and software and provide functions which may be combined in a single element or multiple elements.

The functions of the various elements shown in the FIGS. can be provided through the use of dedicated hardware as well as hardware capable of executing software in association with appropriate software. When provided by a processor, the functions can be provided by a single dedicated processor, by a single shared processor, or by a plurality of individual processors, some of which can be shared. Moreover, explicit use of the term "processor" or "controller" should not be construed to refer exclusively to hardware capable of executing software, and can implicitly include, without limitation, digital signal processor ("DSP") hardware, read-only memory ("ROM") for storing software, random access memory ("RAM"), non-volatile storage, etc.

Moreover, all statements herein reciting principles, aspects, and embodiments of the invention, as well as specific examples thereof, are intended to encompass both structural and functional equivalents thereof. Additionally, it is intended that such equivalents include both currently known equivalents as well as equivalents developed in the future (i.e., any elements developed that perform the same function, regardless of structure). Thus, for example, it will be appreciated by those skilled in the art that the block diagrams presented herein represent conceptual views of illustrative system components and/or circuitry embodying the principles of the invention. Similarly, it will be appreciated that any flow charts, flow diagrams and the like represent various processes which may be substantially represented in computer readable storage media and so executed by a computer or processor, whether or not such computer or processor is explicitly shown.

Furthermore, embodiments of the present invention can take the form of a computer program product accessible from a computer-usable or computer-readable storage medium providing program code for use by or in connection with a computer or any instruction execution system. For the purposes of this description, a computer-usable or computer readable storage medium can be any apparatus that may include, store, communicate, propagate, or transport the program for use by or in connection with the instruction execution system, apparatus, or device. The medium can be an electronic, magnetic, optical, electromagnetic, infrared, or semiconductor system (or apparatus or device) or a propagation medium. Examples of a computer-readable medium include a semiconductor or solid state memory, magnetic tape, a removable computer diskette, a random access memory (RAM), a read-only memory (ROM), a rigid magnetic disk and an optical disk. Current examples of optical disks include compact disk - read only memory (CD-ROM), compact disk - read/write (CD-R/W) and DVD.

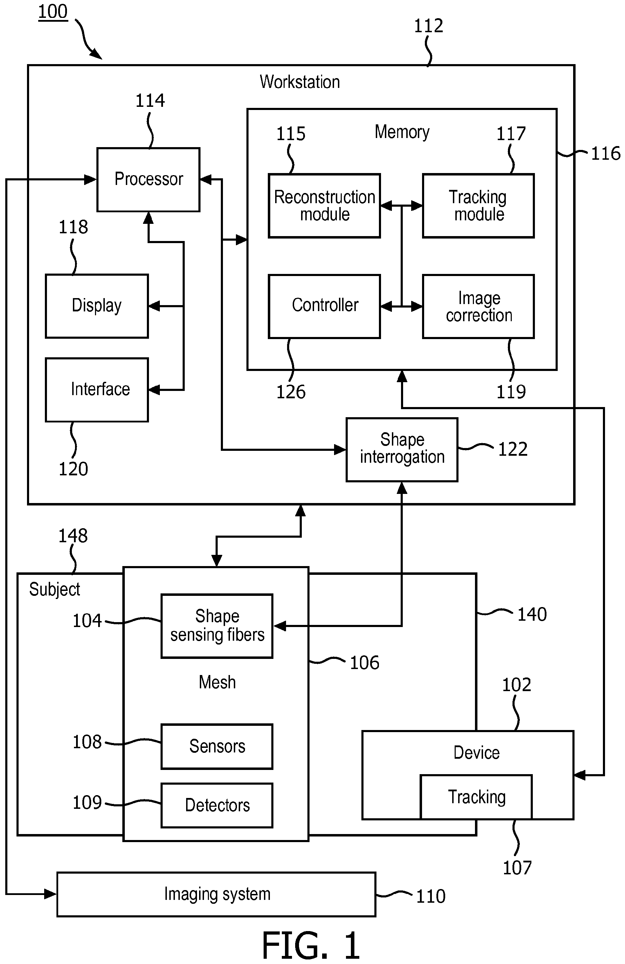

Referring now to the drawings in which like numerals represent the same or similar elements and initially to FIG. 1, a system 100 for performing a medical procedure is illustratively depicted. System 100 may include a workstation or console 112 from which a procedure is supervised and managed. Procedures may include any procedure including but not limited to biopsies, ablations, injection of medications, etc. Workstation 112 preferably includes one or more processors 114 and memory 116 for storing programs and applications. It should be understood that the function and components of system 100 may be integrated into one or more workstations or systems.

Memory 116 may store a reconstruction module 115 configured to interpret electromagnetic, optical, acoustic, etc. feedback signals from a sensitized flexible mesh 106. The mesh 106 may include fiber sensors, optical, acoustic, electrical or electromagnetic markers or sensors, etc. embedded therein with known geometry or with a geometery that is initialized before use. A shape interrogation console 122 measures the marker/sensor distribution over the surface of interest and supplies feedback about calibration / reference sections and measurement sections to the reconstruction module 115. The shape interrogation module 122 sends and receives light to/from optical fiber sensors 104. The optical fiber sensors 104 are weaved or otherwise integrated into mesh 106 in a pattern that allows for stretching of the underlying textile substrate while accounting for the fact that the overall fiber sensor length in the textile can change only minimally (e.g., a 2D spiral pattern or 2D sinusoidal pattern embedded within the flexible membrane). The fibers 104 are locally anchored at control points to provide a strain in the fiber during the flexure of the subject 148. Several control points can constrain the fiber in all degrees of freedom relative to the mesh, e.g., at the fiber tip, whereas others can allow for a sliding degree of freedom so that the fiber can slide freely relative to the mesh pattern to accommodate any overall path length changes in the patterned structure as the mesh deforms.

The reconstruction module 115 may include the capability of receiving multiple inputs from multiple devices or systems to interpret an event or dynamic occurrence during a medical procedure, diagnostic test, etc. A medical imaging device 110 and/or a tracking module 117 may also be included and may provide additional feedback to the reconstruction module 115. The reconstruction module 115 is configured to use the signal feedback (and any other feedback) to account for errors or aberrations related to dynamic changes of a patient's body.

In one embodiment, a subject 148 or a region of interest 140 on the subject 148 is covered or constrained by the flexible mesh 106. The flexible mesh 106 may include a fabric or netting configured to stretch corresponding with movement or flexure of the subject 148 or the region of interest 140. The mesh 106 may include one or more sensors 108 and/or detectors 109 distributed within and throughout the mesh 106 that may include technologies other than optical fiber sensing (e.g., electrodes, trackers, etc.). The shape sensing fibers 104, the sensors 108 and/or the detectors 109 are configured to relay positional, temperature, electric field information, shape information, etc. back to the reconstruction module 115. For example, the mesh 106 may be disposed over a mid-section of a patient (148) such that during a breathing cycle sensors 108/detectors 109 sense the dynamic shape changes of the abdomen or chest. This information may be interpreted using the reconstruction module 115 which computes distances or changes in distances between nodes or positions in the mesh 106. The mesh deflections may then be employed to account for breathing in images taken by an imaging device 110 or assist in the timing of an action during a medical procedure (e.g., inserting a device on an exhale, etc.), or any other event or action that needs compensation for dynamic changes.

It should be understood that the mesh 106 may be applied to any portion of the subject's anatomy to collect dynamic shape/position data. For example, the mesh 106 may be placed over the arms, legs, abdomen, chest, neck, head, or combinations thereof. In addition, the mesh 106 may be made adjustable to be configured for different sized subjects, different sized appendages, etc.

The mesh 106 may be employed during a medical procedure to assist a clinician in performing the procedure. For example, a medical device 102 may include, e.g., a needle, a catheter, a guide wire, an endoscope, a probe, a robot, an electrode, a filter device, a balloon device or other medical component, etc. Workstation 112 may include a display 118 for viewing internal images of the subject 148 using the imaging system 110 in addition to processing and computational components that automatically perform registration and fusion of shape sensing information relative to imaging or other clinical data. The imaging system 110 may include imaging modalities, such as, e.g., ultrasound, photoacoustics, a magnetic resonance imaging (MRI) system, a fluoroscopy system, a computed tomography (CT) system, positron emission tomography (PET), single photon emission computed tomography (SPECT), or other system. Imaging system 110 may be provided to collect real-time intraoperative imaging data. The imaging data may be displayed on display 118. Display 118 may permit a user to interact with the workstation 112 and its components and functions. This is further facilitated by an interface 120 which may include a keyboard, mouse, a joystick or any other peripheral or control to permit user interaction with the workstation 112.

During the procedure, images collected by the imaging device 110 may need to be compensated for due to three-dimensional (3D) dynamic patient movements, breathing etc. Such movements may be accounted for in the displayed images to provide a steadier real-time display image of the subject or region of interest. One or more tracking devices or cameras 107 may be incorporated into the device 102, so tracking/imaging information can be detected. The tracking devices 107 may include electromagnetic (EM) trackers, fiber optic tracking, robotic positioning systems, cameras, etc.

Reconstruction module 115 may employ compensation models or position algorithms to compute discrepancies or account for dynamic changes between images due to dynamic changes. A digital rendering of the region of interest 140 and/or the device 102 (using feedback signals) can be displayed with aberrations and errors accounted for due to the dynamic changes. The digital rendering may be generated by an image correction module 119.

In one embodiment, the system 100 acquires data to generate tomographic images of the human body and performs radiation therapy adaptive to patient motion. Reconstruction and optimization algorithms may be employed by the reconstruction module 115 to take into account variable measurement geometries derived from shape tracking of the mesh 106. This information can be applied to a controller module 126 or other device to condition signals to adapt for the dynamic changes. In one example, the controller module 126 may generate control signals to control various controllers, sensors, radiation sources/beams, etc. in accodance with the dynamic changes to improve data collection, feedback or execution of the activities taking place during a procedure or study. The feedback loop between the shape reconstruction computed by the reconstruction module 115 dynamically provides information about the 3D shape of the flexible mesh 106 which may be employed in administering medication, chemotherapy, radiation, measuring muscle activity, etc. by the clinical system.

In this way, the optical fiber sensing 104 provides additional information about the second modality nodes, electrodes, tracking devices, sources, etc. that are disposed in the mesh 106. Relative distances of the nodes (e.g., sensors 108, detectrors 109, etc.) are known with respect to time, and this data may be employed to make more accurate predictions, estimate future doses/treatments, determine accuracy of measurements, etc.

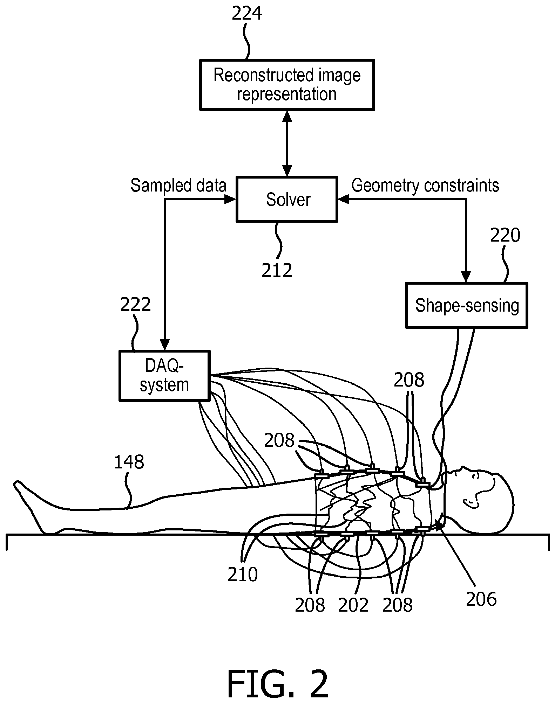

Referring to FIG. 2, a vest 202 is formed from a mesh 206 which includes the features of mesh 106. The vest 202 is preferably flexible with a snug fit over the subject 148. Sensing fibers 210 are integrated in the vest 202 and are employed to determine a shape of the chest of the subject 148. A shape-sensing module 220 interrogates the fibers 210, and this information is used in a solver module 212 which models real geometry of the subject 148 for reaching optimal solutions for, e.g., an inversion problem. The sensing fiber(s) 210 may include a single optical fiber integrated into the vest 202 that spirals around the subject's body and hence delivers a sufficient picture of the geometry. The sensing fibers 210 may also include multiple fibers integrated into the vest 202.

The sensing fibers 210 may include one or more fiber optic Bragg gratings (FBG), which are a segment of an optical fiber that reflects particular wavelengths of light and transmits all others. This is achieved by adding a periodic variation of the refractive index in the fiber core, which generates a wavelength-specific dielectric mirror. A FBG can therefore be used as an inline optical filter to block certain wavelengths, or as a wavelength-specific reflector.

A fundamental principle behind the operation of a FBG is Fresnel reflection at each of the interfaces where the refractive index is changing. For some wavelengths, the reflected light of the various periods is in phase so that constructive interference exists for reflection and consequently, destructive interference for transmission. The Bragg wavelength is sensitive to strain as well as to temperature. This means that FBGs gratings can be used as sensing elements in fiber optical sensors. In a FBG sensor, a shift in the Bragg wavelength, $\Delta\lambda_B$ is caused,

and the relative shift in the Bragg wavelength, $\Delta\lambda_B/\lambda_B$, due to an applied strain ($\varepsilon$) and a change in temperature ($\Delta T$) is approximately given by:

$$\frac{\delta\lambda_B}{\lambda_B} = C_s\varepsilon + C_T\Delta T$$

.

The coefficient $C_s$ is called the coefficient of strain and its magnitude is usually around 0.8 x 10$^{-6}$ / $\mu\varepsilon$ or in absolute quantities about 1 pm/ $\mu\varepsilon$. The coefficient $C_T$ describes the temperature sensitivity of the sensor and is made up of the thermal expansion coefficient and the thermo optic effect. Its value is around 7x10$^{-6}$/K (or as an absolute quantity 13 pm/K).

One of the main advantages of the technique is that various sensor elements can be distributed over the length of a fiber. Incorporating 3 or more cores with various sensors (gauges) along the length of a fiber that is embedded in a structure allows for evaluation of the curvature of the structure as a function of longitudinal position and hence for the 3 dimensional form of such a structure to be precisely determined, i.e., the volume constraining an ill-posed problem.

As an alternative to FBGs, the inherent backscatter in conventional optical fibers can be exploited. One such approach is to use Rayleigh scatter in standard single-mode communications fiber. Rayleigh scatter occurs as a result of random fluctuations of the index of refraction in the fiber core. These random fluctuations can be modeled as a Bragg grating with a random variation of amplitude and phase along the grating length. By using this effect in 3 or more cores running within a single length of multicore fiber, the 3D shape and dynamics of the surface of interest would be trackable.

In one embodiment, the vest 202 may include an electrode vest for body potential surface maps (BPSM). The BPSM vest 202 includes a plurality of electrode sensors/detectors 208 included therein. In this embodiment, the sensors/detectors 208 are connected with a data acquistion system 222 to collect positional information of the body surface. In this setup, the sampled data collected from the electrodes 208 can be correlated with the geometric constraints derived from the shape sensing module to provide a more accurate depiction of the dynamics of the subject. In this way, dynamic changes of shape during, e.g., breathing, etc. could be accounted for in the solver module 212 and correlated to images to provide a reconstructed image representation 224, which accounts for any sensed movement in the subject. The same set up may be employed for temperature sensing, electric field sensing, vibrational studies, etc. In this way, a dynamic (moving) or static digital rendering of the body or subject is provided along with other real-time measurements to provide a complete set of information for analysis or study.

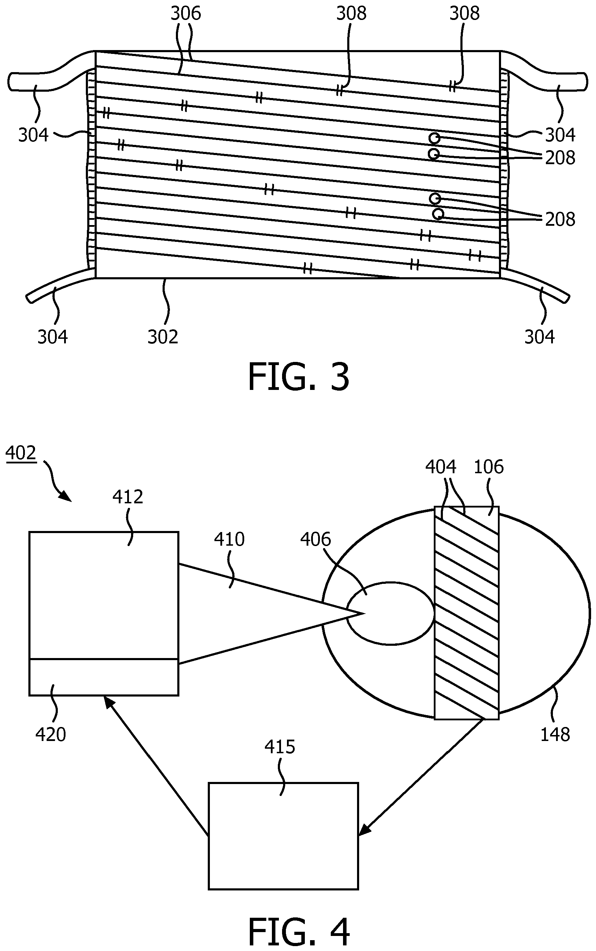

Referring to FIG. 3, in another embodiment, the mesh 106, 206 may be fabricated into a form of clothing, e.g., a pair of trunks, a bra, a skull cap, a sock, a glove, etc. The clothing may include elastic, Spandex ™ or other form of elastic clothing. For purposes of illustration, a garment 302 may be configured for diffuse optical tomography (DOT) of the breast. The garment 302 includes adjustment mechanisms 304 to be adaptively sized to snuggly fit a patient. The adjustment mechanisms 304 may include hook and loop connectors, buckles, elastic bands, zippers, snaps or any other devices for adjusting a size. Optical sensing fibers 306 are disposed within the fabric of the garment 302 and optical signals are sent and received to locations in the garment 302 which are in direct contact with anatomy tissue. The same or different fibers may be employed for sensing and for optical tomography (e.g., depending on the application multimode fibers may be needed to increase signal strength instead of single mode fibers used for optical shape sensing).

In one case, the anatomy tissue is the breast, and the fibers can be used for shape sensing / tracking of the measurement geometry (Raleigh scattering), or, in a multi-core fiber setup, additional core fibers which may include Bragg gratings and can be used as a means for shape estimation. Other geometry tracking techniques may be considered for determining the shape of the anatomically adapted garment 302. The optical fibers 306 are preferably locally bound or connected to the garment 302 using connections 308. This may be achieved by cross-stitching, by using optical fibers that are angled, are woven in or possess a serpentine arrangement in the garment or by employing an adhesive, etc.

It should be noted that other devices or medications may be employed in the garment to create a synergy between the geometry data collected using the optical fibers 306 and the other systems. In one example, the garment 302 may include radiation sources integrated therein that are disposed around the breast, and the movement of the breast may be employed to evaluate radiation dosage actually received in areas of the breast. Other applications are also contemplated.

Referring to FIG. 4, a system 402 is shown for radiation therapy. Subject 148 has a lesion or tumor 406 that needs to be treated with radiation therapy. To reduce the risk of destroying healthy tissue, accounting for patient dynamics is an important consideration. By employing mesh 106 with optical sensing fibers 404, patient dynamics can be accounted for. A radiation beam 410 from a source 412 can be pulsed or otherwise timed in accordance with the dynamics measured using the sensing fibers 404. In another embodiment, a controller 415 controls a device 420, such as a servo, an actuator, a digital signal processor, a shutter, etc., which may be employed to move the source 412 or direct/control the beam 410 in accordance with the patient dynamics data. This may result in controlling the timing of radiation pulses and/or aiming the pulses to more accurately hit the target tissue (e.g., tumor 406). The patient dynamics data can be used to drive patient models for more advanced system control.

While the present principles have been described in accordance with illustrative nonlimiting examples (e.g., imaging techniques, radiation therapy, etc.), other applications can benefit from the inventive concepts described herein, e.g., the present principles may be employed in solving ill-posed problems. Ill-posed problems can be described as problems that have a solution deduced from final data (i.e., the solution is highly sensitive to changes in the final data). The present principles may be employed in solving inverse problems, which include a general framework that is used to convert observed measurements into information about a physical object or system of interest, for example, the solution is derived to match the data. The present principles may be employed in temperature mapping, neuronal imaging (e.g., magnetoencephalography / electroencephalography (MEG/EEG), measurement of muscle flexure, etc.

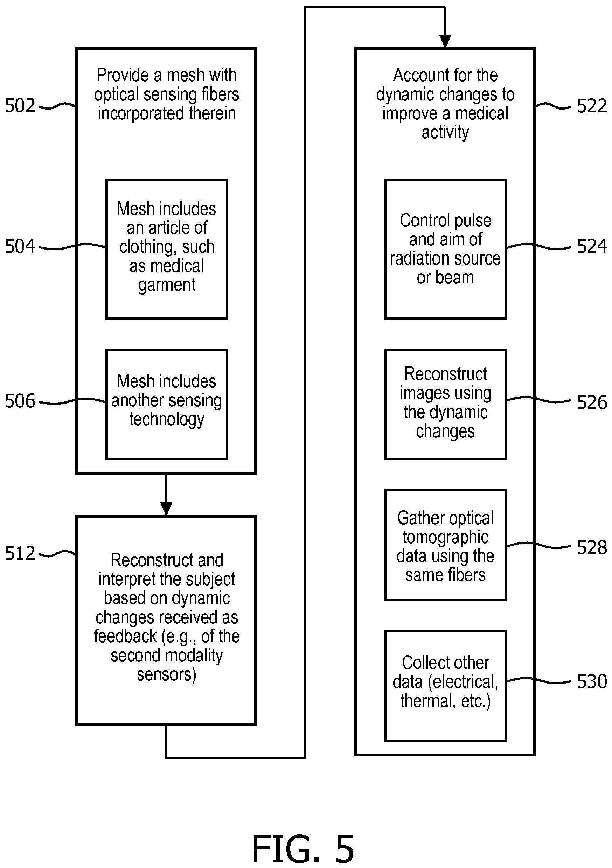

Referring to FIG. 5, a method for measuring dynamic movement of a subject is illustratively shown in accordance with illustrative embodiments. In block 502, a mesh is provided which is configured to flexibly and snuggly fit over at least a portion of the subject, e.g., an arm, leg, chest, etc. The mesh includes one or more shape sensing optical fibers disposed therein. The optical fibers are woven into the fabric of the mesh and are preferably attached to the mesh fabric such that flexing the mesh imparts a strain in the fiber. The optical fibers may be tied to the mesh, glued or otherwise coupled to the mesh to permit flexure but also to constraint the motion of the portion of the subject. In block 504, the mesh may include an article of clothing, a medical garment configured to perform position sensing with detectors disposed on the medical garment and wherein the one or more shape sensing optical fibers are employed to improve the position sensing of sensors, detectors, etc. or otherwise measure dynamic changes in a subject.

In block 506, in a preferred embodiment, the mesh includes another sensing modality used in conjunction with the fiber optic sensing. For example, sensors or electrodes may be employed which are surrounded by or integrated with the fiber optic sensors. In this way, feedback regarding the movement or displacement of the patient and/or the sensors or electrodes is enhanced by the fiber optic sensing data.

In block 512, a shape of the portion of the subject is reconstructed by employing feedback signals received from the one or more shape sensing fibers, and the dynamic changes in a shape and position of the mesh are interpreted based on the feedback signals.

In block 522, the dynamic changes measured are accounted for to improve a medical activity on the subject, e.g., treatment, diagnoses, analysis of a second modality incorporated in the mesh. Examples may include the following. In block 524, a pulse and/or aim of a radiation beam for treating the subject is controlled in accordance with the dynamic changes. The beam is modulated in time or moved to keep its focal point on target to destroy a lesion or tumor without destroying healthy tissue. In block 526, an image or images are reconstructed based upon the dynamic changes to provide a more accurate measurement or rendering of the image without unwanted artifacts due to movement of the subject. In block 528, optical tomographic data or other data of the subject may be gathered using the same (or different) shape sensing optical fibers. In block 530, electric field data, thermal data or other data is collected. The data collected is matched with or accounted for in view of the shape sensing optical fiber information. For example, one or more shape sensing optical fibers may be used in conjunction with electrical or thermal measurements to perform electrical impedance tomography or body surface potential / temperature mapping.

In interpreting the appended claims, it should be understood that:

a) the word "comprising" does not exclude the presence of other elements or acts than those listed in a given claim;

b) the word "a" or "an" preceding an element does not exclude the presence of a plurality of such elements;

c) any reference signs in the claims do not limit their scope;

d) several "means" may be represented by the same item or hardware or software implemented structure or function; and

e) no specific sequence of acts is intended to be required unless specifically indicated.

Having described preferred embodiments for systems for dynamic constraining with optical shape sensing (which are intended to be illustrative and not limiting), it is noted that modifications and variations can be made by persons skilled in the art in light of the above teachings. The invention is as set forth in the appended claims.

## Claims

1. A system for measuring dynamic movement of a subject, comprising:

a mesh (106) configured to flexibly and snuggly fit over at least a portion of the subject (140), the mesh including one or more shape sensing optical fibers (104) disposed therein and arranged to provide light reflected back from within the fibers, the mesh further including a second feedback modality of measurement other than optical fiber sensing having sensors (108) or detectors (109) incorporated and distributed within and throughout the mesh such that the one or more shape sensing optical fibers are arranged to monitor movement of the sensors or detectors on the subject; and
a reconstruction module (115) coupled to the shape sensing fibers to receive feedback signals based on the reflected light from the shape sensing fibers and to interpret dynamic changes in a shape and position of the sensors or detectors based on the feedback signals, the reconstruction module accounting for the dynamic changes to improve a medical activity on the subject.

2. The system as recited in claim 1, wherein the reconstruction module (115) is configured to control a pulse and aim radiation for treating the subject.

3. The system as recited in claim 1, wherein the reconstruction module (115) is configured to reconstruct an image based upon the dynamic changes.

4. The system as recited in claim 1, wherein the sensors (108) or detectors (109) includes electrodes in direct contact the subject.

5. The system as recited in claim 1, wherein the one or more shape sensing optical fibers (104) include a single optical fiber wrapped in the mesh.

6. The system as recited in claim 1, wherein the mesh (106) includes a medical garment configured to perform position sensing using the sensors or detectors disposed on the medical garment and wherein the one or more shape sensing optical fibers are employed to verify and improve the position sensing.

7. The system as recited in claim 1, wherein the one or more shape sensing optical fibers (104) are employed to gather optical tomographic data of the subject.

8. The system as recited in claim 1, wherein the one or more shape sensing optical fibers (104) are employed in conjunction with electrical measurements to perform electrical impedance tomography.

9. The system as recited in claim 1, wherein the one or more shape sensing optical fibers (104) are employed in conjunction with thermal measurements to perform body surface potential / temperature mapping.

## Patentansprüche

1. System zur Messung einer dynamischen Bewegung eines Objekts, umfassend:

ein Mesh (106), das so ausgeführt ist, dass es flexibel und perfekt über zumindest einen Teil des Objekts (140) passt, wobei das Mesh eine oder mehrere optische Shape-Sensing-Fasern (104) enthält, die darin angeordnet und so vorgesehen sind, dass sie von innerhalb der Fasern rückreflektiertes Licht bereitstellen, wobei das Mesh weiterhin eine zweite Feedback-Modalität einer anderen Messung als der faseroptischen Messung mit Sensoren (108) oder Detektoren (109) enthält, die in dieses integriert und innerhalb und über das gesamte Mesh so verteilt sind, dass die eine oder mehrere optische Shape-Sensing-Fasern so angeordnet sind, dass sie die Bewegung der Sensoren oder Detektoren auf dem Objekt überwachen; sowie ein mit den Shape-Sensing-Fasern gekoppeltes Rekonstruktionsmodul (115), um Rückführungssignale aufgrund des von den Shape-Sensing-Fasern reflektierten Lichts zu empfangen und dynamische Änderungen in einer Form und Position der Sensoren oder Detektoren aufgrund der Rückführungssignale zu interpretieren, wobei das Rekonstruktionsmodul die dynamischen Änderungen erfasst, um eine medizinische Aktivität auf dem Objekt zu verbessern.

2.  System nach Anspruch 1, wobei das Rekonstruktionsmodul (115) so konfiguriert ist, dass es eine Impuls- und Zielstrahlung zur Behandlung des Objekts steuert.

3.  System nach Anspruch 1, wobei das Rekonstruktionsmodul (115) so konfiguriert ist, dass es ein auf den dynamischen Änderungen basierendes Bild rekonstruiert.

4.  System nach Anspruch 1, wobei die Sensoren (108) oder Detektoren (109) Elektroden in direktem Kontakt mit dem Objekt enthalten.

5.  System nach Anspruch 1, wobei die eine oder mehrere optische Shape-Sensing-Fasern (104) eine in das Mesh eingehüllte, einzelne optische Faser enthalten.

6.  System nach Anspruch 1, wobei das Mesh (106) ein medizinisches Bekleidungsstück enthält, das so vorgesehen ist, dass es eine Positionserfassung unter Verwendung der auf dem medizinischen Bekleidungsstück angeordneten Sensoren oder Detektoren vornimmt, und wobei die eine oder mehrere optische Shape-Sensing-Fasern zur Verifizierung und Verbesserung der Positionserfassung eingesetzt werden.

7.  System nach Anspruch 1, wobei die eine oder mehrere optische Shape-Sensing-Fasern (104) zur Erfassung optischer, tomographischer Daten des Objekts eingesetzt werden.

8.  System nach Anspruch 1, wobei die eine oder mehrere optische Shape-Sensing-Fasern (104) in Verbindung mit elektrischen Messungen zur Durchführung einer elektrischen Impedanz-Tomographie eingesetzt werden.

9.  System nach Anspruch 1, wobei die eine oder mehrere optische Shape-Sensing-Fasern (104) in Verbindung mit thermischen Messungen zur Durchführung von Body-Surface-Potential- / Temperatur-Mapping eingesetzt werden.

**Revendications**

1.  Système permettant de mesurer un mouvement dynamique d'un sujet, comprenant :

    une maille (106) configurée pour s'adapter de façon souple et parfaite sur au moins une partie du sujet (140), la maille comprenant une ou plusieurs fibres optiques de détection de forme (104) disposées à l'intérieur et agencées pour fournir de la lumière réfléchie en retour à partir de l'intérieur des fibres, la maille comprenant en outre une seconde modalité de rétroaction de mesure autre que la détection de fibre optique ayant des capteurs (108) ou des détecteurs (109) incorporés et répartis à l'intérieur et dans toute la maille de telle sorte que l'une ou plusieurs fibres optiques de détection de forme soient agencées pour surveiller le mouvement des capteurs ou des détecteurs sur le sujet ; et un module de reconstruction (115) couplé aux fibres de détection de forme pour recevoir des signaux de rétroaction basés sur la lumière réfléchie à partir des fibres de détection de forme et pour interpréter des changements dynamiques d'une forme et d'une position des capteurs ou des détecteurs sur la base des signaux de rétroaction, le module de reconstruction tenant compte des changements dynamiques pour améliorer une activité médicale sur le sujet.

2.  Système selon la revendication 1, dans lequel le module de reconstruction (115) est configuré pour commander une impulsion et diriger un rayonnement permettant de traiter le sujet.

3.  Système selon la revendication 1, dans lequel le module de reconstruction (115) est configuré pour reconstruire une image sur la base des changements dynamiques.

4.  Système selon la revendication 1, dans lequel les capteurs (108) ou les détecteurs (109) comprennent des électrodes en contact direct avec le sujet.

**5.** Système selon la revendication 1, dans lequel l'une ou plusieurs fibres optiques de détection de forme (104) comprennent une seule fibre optique enveloppée dans la maille.

**6.** Système selon la revendication 1, dans lequel la maille (106) comprend un vêtement médical configuré pour effectuer une détection de position à l'aide des capteurs ou des détecteurs disposés sur le vêtement médical et dans lequel l'une ou plusieurs fibres optiques de détection de forme sont employées pour vérifier et améliorer la détection de position.

**7.** Système selon la revendication 1, dans lequel l'une ou plusieurs fibres optiques de détection de forme (104) sont employées pour recueillir des données tomographiques optiques du sujet.

**8.** Système selon la revendication 1, dans lequel l'une ou plusieurs fibres optiques de détection de forme (104) sont employées conjointement avec des mesures électriques pour effectuer une tomographie par impédance électrique.

**9.** Système selon la revendication 1, dans lequel l'une ou plusieurs fibres optiques de détection de forme (104) sont employées conjointement avec des mesures thermiques pour effectuer un mappage potentiel de surface corporelle/température.

FIG. 1

FIG. 2

FIG. 3

FIG. 4

**FIG. 5**

**EP 2 717 774 B1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 20110054303 A1